# EUROPEAN PATENT APPLICATION

(11) **EP 4 755 414 A2**
(43) Date of publication of application: **10.06.2026**
(21) Application number: 26171241.8
(22) Date of filing: 04.12.2020
(51) Int. Cl.: A61M 5/00

(54) **SYRINGE AND TIGHTNESS TESTING METHOD**

(30) Priority: 05.12.2019 EP 19213836
(62) Divisional of application: 20816243.8
(71) Applicant: F. Hoffmann-La Roche AG, 4070 Basel (CH)
(72) Inventor: Cammish, Neil B., Manchester, M25 1EJ (GB); Hemminger, Markus, 4070 Basel (CH)
(74) Representative: Latscha Schöllhorn Partner AG

(57) **Abstract**

A method of testing tightness between a stopper (3) and a plunger (4) in a syringe (1), wherein the stopper (3) has a distal face (31), a proximal face (32) and an internal cavity (33) opening at the distal face (31), and the syringe (1) has a barrel (2) with a hollow interior, an orifice (21) and an opening (22) opposite to the orifice (21), the method comprising: opening the cavity (33) of the stopper (3) to the proximal face (32), setting the stopper (3) through the opening (22) of the barrel (2) into a hollow interior of the barrel (2) such that the distal face (31) of the stopper (3) is directed towards the plunger (4), coupling a gas detector to the opening (22) of the syringe barrel (2), and providing a gas through the orifice (21; 210; 219) of the barrel (2; 20; 29).

## Description

### Technical Field

The present invention relates to a syringe and more particularly to a method of testing tightness between a stopper and a plunger of a syringe.

Such syringes comprising (i) a barrel having a hollow interior, an orifice and an opening opposite to the orifice, (ii) a stopper arranged in the hollow interior of the barrel thereby defining a sealed chamber in the interior of the barrel, wherein the stopper is displaceable in the interior of the barrel thereby varying a volume of the chamber, and (iii) a plunger extending through the opening of the barrel into the hollow interior of the barrel, can be used for delivering a drug substance to a patient. In particular, in case the syringe is embodied as prefilled syringe, it can allow for convenient administration of a predefined dose of the drug substance to the patient.

### Background Art

Many pharmaceuticals or drug substances are administered in liquid form. For an efficient administration and efficacy, the liquid drug substance is often delivered parenterally by injection. Thereby, particularly for subcutaneous, intramuscular, intradermal or intravitreal injection, the drug substances are often provided in prefilled syringes (PFS). Such PFS may have staked needles or be capable of accepting a separate connecting needle. In a PFS, the drug substance is provided in the interior of a barrel of the syringe in a solution, suspension or other liquid form ready for administration. A PFS may have the advantage that the user receives a (quasi) ready-to-inject syringe without requiring preparation such as filling the syringe with the drug substance, e.g. by transfer from a vial or the like. The contamination of the drug substance by e.g. particles and microbes, injuries, and/or inappropriate or inconvenient handling during application can thereby be reduced. Also, a PFS may allow self-administration by a patient.

Usually, a PFS comprises a barrel having an open end and a tip with an orifice essentially opposite to the open end, a rubber stopper, a plunger and a needle or needle adaptor on the orifice of the barrel. For example, a specific ophthalmic PFS is described in WO 2014/005728 A1.

Whereas, compared to conventional drug preparation, a PFS can be beneficial in use or administration, it is typically more challenging to manufacture drug substances in a PFS than in other containers such as vials. One aspect which may be of high importance particularly in ophthalmic applications is the external sterilization of the syringe after complete assembly, composition and/or packaging.

In such external sterilization, the external surface of the syringe is sterilized typically using gaseous chemical sterilization. Thereby, in order to prevent the drug substance inside the syringe barrel to be affected, it can be important to prevent that the sterilizing agent enters the sealed interior of the barrel. In particular, ingress of the sterilizing agent should be below the limit provided by health authorities or the International Organization for Standardization (ISO), or must not compromise the drug quality until end of shelf life. For example, in case ethylene oxide (EO) is used as sterilization agent, the European Agency for the Evaluation of Medicinal Products (EMEA) specifies in EMEA/CVMP/271/01 guidance a limit of 1 µg/mL EO and 50 µg/mL ethylene chlorihydrin (ECH). Or, ISO10993-7 specifies a limit of 0.5 µg EO/IOL/24 hr and 1.25 µg EO/IOL, which interprets 0.5 µg EO/eye/24 hr and 1.25 µg EO/eye as well as 2.0 µg ECH/IOL/24 hr and 5.0 µg EO/IOL, which interprets 2.0 µg EO/eye/24 hr and 5.0 µg EO/eye.

A problem occurring in such external surface sterilization of syringes can be that the sterilizing agent is not capable of sufficiently reaching all parts and portions of a syringe other than the drug substance inside the barrel. Accessing spaces and areas in the region of the plunger coupled to or abutting the stopper typically is difficult. In particular, when widespread stoppers having an internal cavity are used, the cavity itself may be difficult to be reached by the sterilizing agent. More specifically, often the plunger is provided within the cavity of the stopper or abutting it such that entering of the sterilizing agent into the cavity can be hindered. This may result in contaminants such as viable organisms residing in the cavity from where at a later stage they may escape the syringe and lower safety of using the syringe. Solid stoppers without cavity may not be practical in certain applications, e.g., due to machinability challenges and/or not being available as a commercial solution. A further problem is that assembly force of the plunger to the stopper is limited so as to avoid stopper movement and/or compression of the syringe fluid contents during or after plunger assembly.

Therefore, there is a need for a system or a syringe capable of lowering the risk of contamination in use or application.

### Disclosure of the Invention

According to the invention this need is settled by a method as it is defined by the features of independent claim 1. Preferred embodiments are subject of the dependent claims.

In one aspect, the disclosure is a syringe comprising a barrel, a stopper and a plunger. The barrel has a hollow interior, an orifice and an opening opposite to the orifice. The stopper is arranged in the hollow interior of the barrel thereby defining a sealed chamber in the interior of the barrel. The stopper is displaceable in the interior of the barrel thereby varying a volume of the chamber. In use, the plunger extends through the opening of the barrel into the hollow interior of the barrel. The stopper can expel the liquid out of the barrel via the orifice when being moved towards the orifice by the plunger such that the volume of the chamber is reduced. The plunger has a distal end outside of the barrel and a proximal end inside the hollow interior of the barrel. The stopper has a distal face directed towards the plunger, a proximal face directed towards the chamber and an internal cavity opening at the distal face. The syringe is equipped with a sealing structure sealing the cavity of the stopper such that the cavity of the stopper is microbiologically sealed.

In the context of the present invention, the term "syringe" relates to all kind of syringes in the literal sense such as single chamber syringes, double chamber syringes or multi chamber syringes. The syringe can be prefilled, partially filled or empty. Also the can be provided with a staked needle or an adapter to which a needle or a similar administration duct can be coupled. Besides syringes in the literal sense, the term "syringe" as used herein also covers similar devices or containments such as, particularly, cartridges.

The term "proximal" as used herein refers to a portion, an extremity or a component located closest to or a direction oriented towards a medicament or drug delivery site, when the syringe is in use. Thus, the proximal direction can be a direction towards the body or person to which the syringe is intended to be applied. For example, in embodiments of syringes having a needle intended to be pierced in the body or person and the plunger to be pushed for delivering a medicament through the needle, the proximal end of the syringe is formed by a tip of the needle. The proximal direction can be the direction towards an end of the orifice of the barrel or a location of the skin of the patient penetrated by a needle upon delivery of the medicament or drug substance to the patient.

Conversely, the term "distal" is used to refer to a portion, an extremity or a component located furthest away from or a direction oriented away from a medicament delivery site when the syringe is in use. Thus, a distal direction can be a direction oriented away from a body or person to which the syringe is to be applied. For example, in use, the distal end of the syringe can be the end of the plunger where a thumb of an operator is placed for forwarding the plunger in order to deliver the medicament. Typically, proximal and distal are opposite directions.

The term "tight" as used in connection with the stopper and the plunger can particularly relate to gas-tight or, more specifically, tight for contaminations such as deposits of organic (e.g. microorganisms or endotoxins). Advantageously, tightness is sufficient to prevent escaping of potential microbes and/or endotoxins from the stopper cavity.

Preferably, a liquid is arranged in the chamber of the barrel. Like this, the syringe can be a prefilled syringe. In a particularly advantageous embodiment, the syringe is an ophthalmic prefilled syringe, wherein the drug substance is an ophthalmic drug substance.

The barrel of the syringe can have a main portion which is essentially cylindrical. Particularly, the main portion can have the shape of a hollow circular cylinder. The barrel can be made of any suitable material and for most pharmaceutical applications of a sterilisable inert material such as an appropriate rigid plastic material or, particularly, glass. Glass barrels may be beneficial for manufacturing, sterility, inertness and stability reasons. The opening of the barrel can be an opening over the complete diameter of the interior of the barrel. The orifice can be embodied in a tip or spout formed in the end of the barrel opposite to the opening. In particular, the orifice can have a channel in the tip having a reduced diameter compared to the diameter of the interior of the barrel. The orifice can be dimensioned to allow the liquid to be expelled when the volume of the chamber in the barrel is reduced by advancing the stopper via the plunger.

The term "drug" as used herein relates to a therapeutically active agent, also commonly called active pharmaceutical ingredient (API), as well as to a combination or plurality of such therapeutically active substances. The term also encompasses diagnostic or imaging agents, like for example contrast agents such as MRI contrast agents, tracers such as PET tracers, and hormones, that need to be administered in liquid form to the patient.

The term "drug substance" as used herein relates to a drug as defined above formulated or reconstituted in a form that is suitable for administration to a patient. For example, besides the drug, a drug substance may additionally comprise an excipient and/or other auxiliary ingredients. A particularly preferred drug substance in the context of the invention is a drug solution, in particular a dug solution for injection.

Typically, the liquid inside the chamber of the syringe barrel is a drug substance. In case of a double chamber prefilled syringe one chamber may comprise the drug substance which has to be reconstituted for drug administration by a diluent which is contained in a second chamber. Alternatively, the first and second chamber may contain two different drug substances which have to be mixed before drug administration. In particular, the syringe may house a specific dosage of the drug substance to be administered when being injected.

The term "drug product" as used herein relates to a finished end product comprising a drug substance or a plurality of drug substances. In particular, a drug product may be a ready to use product having the drug substance in an appropriate dosage and/or in an appropriate form for administration. For example, a drug product may include an administration device such as the syringe in form of a prefilled syringe or the like.

The term "sterilizing agent" as used herein relates to any liquid, gaseous or vaporized substance capable for externally or external sterilizing the PFS surface. For example, the sterilizing agent can be or comprise ethylene oxide (EO), hydrogen peroxide (H₂O₂), steam, vaporized hydrogen peroxide (VHP), vaporized peracetic acid (VPA), or nitrogen dioxide.

Thereby, the term "sterilize" relates to bringing a structure or element such as the PFS in a sterile state. The term "sterile" as used herein relates to a maximum contamination rate allowing the PFS or another element to be used in an intended application. For example, it can relate to a state of the PFS conforming with the requirements and guidance according to the Standard ST67 of the American National Standards Institute (ANSI) and the Association for the Advancement of Medical Instrumentation (AAMI), i.e. to ANSI/AAMI ST67. More particularly, a sterility assurance level (SAL) value of 10⁻⁶ can be used for products to be labeled as sterile as specified in ANSI/AAMI ST67.

Thus, by means of the sterilizing agent or of the sterilization a situation free of any viable organisms can be achieved. In particular, sterilization can relate to a validated process used to render a product essentially free of viable organisms. In such a sterilization process, the increase of the microbiological death can be described by an exponential function. Therefore, the number of microorganisms which survive a sterilization process can be expressed in terms of probability.

The stopper may be made of an inert and resiliently deformable material such as rubber or silicone. In particular, it can be embodied to seal the chamber in the interior of the barrel containing fluid(s), or a solid and a fluid. Further, it has the distal face which may be oriented towards a liquid in the chamber and the distal face orientated towards the plunger. The outer circumference of the stopper can correspond to the inner circumference of the barrel when fitted. The cavity may be centrally positioned in order to receive the proximal portion of the plunger in a centralized fashion.

The syringe can have a central axis along which the barrel, the stopper and the plunger extend. The syringe or some parts of it such as the barrel can be rotational symmetric about the central axis.

By means of the sealing structure the cavity of the stopper can be closed and sealed. This allows for assuring that any contaminants such as viable organisms cannot escape the stopper cavity at any time of application of the syringe. Thus, if external sterilizations fails to achieve complete sterilization of the cavity of the stopper, safety of the syringe can anyway be provided since contaminants eventually residing in the cavity of the stopper cannot escape due to the sealing structure microbiologically tightly closing the cavity. Like this, the syringe can lower or even eliminate the risk of contaminants escaping the cavity of the stopper after final sterilization or during use of the syringe.

The proximal end of the plunger can form a stopper contact section allowing to provide a suitable transfer of a force from the plunger to the stopper. For example, such transfer may be aimed to be more or less uniform in order to prevent differential deformation of the stopper when forwarding it to expel the liquid.

Thus, after coupling of the plunger with the stopper the embodied component design and materials may guarantee a constant pressing of a plunger rod area against a corresponding stopper area thus creating a microbiologically sealed interface.

In a preferred first embodiment, the proximal end of the plunger has an abutting face and a barb proximally extending from the abutting face, and the cavity of the stopper and the barb upon the proximal end of the plunger are shaped to snap-fit such that the abutting face of the proximal end of the plunger is adjacent to the distal face of the stopper and the stopper is secured to the plunger.

The term "secured" in this context relates to a connection between the stopper and the plunger which is sufficiently strong in order that the stopper and the plunger are not removed from each other in use of the syringe. Thereby, this connection allows that the stopper and the plunger are moved together or conjointly as one single unit. In particularly, by providing the snap-fitting formation embodied by the barb and the cavity, an efficient mechanical coupling can be achieved which allows for establishing a form fit connection.

Thereby, in the first embodiment of the syringe, the barb of the proximal end of the plunger preferably has a (distal) neck portion and a (proximal) head portion, the cavity of the stopper has a (distal) channel portion and an (proximal) interior chamber portion, and the cavity of the stopper and the barb of the proximal end of the plunger are snap-fitted by the channel portion of the cavity of the stopper receiving the neck portion of the barb of the proximal end of the plunger and the interior chamber portion of the cavity of the stopper receiving the head portion of the barb of the proximal end of the plunger. Such arrangement allows for efficiently providing a form fitting connection between the plunger and the stopper and pressing the plunger against the stopper, when being snap-fitted together.

Thereby, preferably, the neck portion of the barb upon the proximal end of the plunger has a first axial length extending between the abutting face of the proximal end of the plunger and the head portion of the barb upon the proximal end of the plunger, the channel portion of the cavity of the stopper has a second axial length extending between the distal face of the stopper and the interior chamber portion of the cavity of the stopper, and the first length of the neck portion of the barb upon the proximal end of the plunger is smaller than the second length of the channel portion of the cavity of the stopper when the stopper is not snap-fitted to the plunger. More specifically, the difference in lengths is particularly given before the stopper is connected to the plunger. By sizing the first and second lengths in that way the sealing structure can be embodied and it can be achieved that the stopper is compressed when the plunger is mounted to the stopper such that the cavity is sealed by the plunger and the connection between plunger and stopper is microbiologically tight and, typically, also gas tight. In particular, the distal face of the stopper can be pressed to the proximal end of the plunger such that the stopper is compressed and tightly connected to the plunger. This allows for efficiently sealing the cavity. Thereby, the first length of the neck portion of the barb upon the proximal end of the plunger preferably is at least 0.3 millimeter (mm) or about 0.5 mm smaller than the second length of the channel portion of the cavity of the stopper. Particularly, when 1 ml or 0.5 ml prefilled syringes are involved, such differences have proved to be appropriate for, on the one hand, assuring tightness of the closing of the cavity and, on the other hand, enabling an efficient connecting or assembling of the plunger and the stopper.

Preferably, the neck portion of the barb upon the proximal end of the plunger has a first compression section, the channel portion of the cavity of the stopper has a second compression section, and the first compression section of the neck portion of the barb of the proximal end upon the plunger is more tapering than the second compression section of the channel portion of the cavity of the stopper.

The term "more tapering" in connection with the compression sections relates to the first compression section of the neck portion is getting thinner or reducing its diameter more than the second compression section of the channel portion along the same length. Thereby, an angle between the shoulder of the first compression section and an axis of the barb can be bigger than an angle between the shoulder of the second compression section and an axis of the channel portion.

Such difference in tapering of the cavity and the barb allows for efficiently embodying the sealing structure and for compressing the stopper between the inside and the outside, e.g. at a beginning of the cavity. Like this, a tight connection between the plunger and the stopper and a sealing of the cavity can efficiently be achieved.

Thereby, the first compression section of the neck portion of the barb upon the proximal end of the plunger preferably is tapering towards the head portion of the barb upon the proximal end of the plunger. The first compression section can be conical. Additionally or alternatively, the second compression section of the channel portion of the cavity of the stopper preferably is tapering towards the interior chamber portion of the cavity of the stopper. The second compression section can also be conical. Particularly, when both sections are conical, it can be achieved that an increasing compression and tightness is provided when the stopper is snap-fitted to the plunger.

Preferably, the syringe or its sealing structure is embodied by comprising a gasket, or a physically or chemically attached elastomeric member such as an overmolded or co-moulded sealing feature made from thermoplastic elastomer or similar resilient material, arranged about the barb upon the proximal end of the plunger, wherein the gasket or member is compressed between the abutting face of the proximal end of the plunger and the distal face of the stopper. Such gasket or member allows for providing additional or alternative tightness between the plunger and the stopper as well as for greater tolerance variability in terms of material hardness and dimensions and lower plunger assembly force lowering the risk that stopper movements are induced by a comparably high assembly force.

Preferably, the distal face of the stopper has a bulge circumferentially extending around the cavity of the stopper opening at the distal face. Such bulge can allow for greater sealing capability for a given assembly force.

In another aspect, the invention is a method of testing tightness between a stopper and a plunger in a syringe. The stopper has a distal face, a proximal face and an internal cavity opening at the distal face, and the syringe has a barrel with a hollow interior, an orifice and an opening opposite to the orifice. The method comprises: (i) opening the cavity of the stopper to the proximal face, e.g., by piercing a stopper front with a needle or cannula or by cutting of a tip of the stopper without impacting sealing characteristics of the stopper, (ii) setting the stopper through the opening of the barrel into a hollow interior of the barrel such that the distal face of the stopper is directed towards the plunger, (iii) coupling a gas detector to the opening of the syringe barrel, and (iv) providing a gas through the orifice of the barrel.

The steps of the method according to the invention do not mandatorily have to be performed in listed sequence or order (i) to (iv). Rather, other sequences of the listed steps are possible as well. A suitable gas which is also reliably detectable can, e.g., be Helium.

The method allows for efficiently testing tightness between the plunger and stopper. More specifically, when providing the gas through the orifice it enters the cavity through the opened proximal face such that it is arranged inside the cavity. If the gas is now not capable of exiting the cavity though the distal side of the stopper, which can be verified by means of the gas detector, the connection between the stopper and the plunger is microbiologically tight. Also, it is possible to rate the tightness by evaluating the amount of gas and/or the gas flow rate detected distally from the stopper.

Preferably, the method further comprises as step of operating the gas detector to detect gas distally from the stopper. Such arrangement allows for efficiently evaluating tightness of the connection between the stopper and the plunger. Thereby, the gas detector preferably measures a concentration of the gas distally from the stopper. By measuring the concentration, it can be achieved that a level of tightness can be evaluated. For example, this allows for comparing different sealing structures and to select the most appropriate one.

Preferably, the method comprises generating a vacuum distally from the stopper. The vacuum can be in a submillibar pressure range. By such vacuum it can be tried to force the gas through the stopper. Thus, tightness can efficiently be tested.

The disclosure further comprises the following examples:
Example 1 is a syringe comprising: a barrel having a hollow interior, an orifice and an opening opposite to the orifice; a stopper arranged in the hollow interior of the barrel thereby defining a sealed chamber in the interior of the barrel, wherein the stopper is displaceable in the interior of the barrel thereby varying a volume of the chamber; and a plunger extending through the opening of the barrel into the hollow interior of the barrel, wherein the plunger has a distal end outside of the barrel and a proximal end inside the hollow interior of the barrel, wherein the stopper has a distal face directed towards the plunger, a proximal face directed towards the chamber and an internal cavity opening at the distal face, and wherein the syringe is equipped with a sealing structure sealing the cavity of the stopper such that the cavity of the stopper is microbiologically sealed.
Example 2 is the syringe of example 1, wherein the proximal end of the plunger has an abutting face and a barb proximally extending from the abutting face, and the cavity of the stopper and the barb of the proximal end of the plunger are shaped to snap-fit such that the abutting face of the proximal end of the plunger is adjacent to the distal face of the stopper and the stopper is secured to the plunger.
Example 3 is the syringe of example 2, wherein the barb of the proximal end of the plunger has a neck portion and a head portion, the cavity of the stopper has a channel portion and an interior chamber portion, and the cavity of the stopper and the barb of the proximal end of the plunger are snap-fitted by the channel portion of the cavity of the stopper receiving the neck portion of the barb of the proximal end of the plunger and the interior chamber portion of the cavity of the stopper receiving the head portion of the barb of the proximal end of the plunger.
Example 4 is the syringe of example 3, wherein the neck portion of the barb of the proximal end of the plunger has a first axial length extending between the abutting face of the proximal end of the plunger and the head portion of the barb of the proximal end of the plunger, the channel portion of the cavity of the stopper has a second axial length extending between the distal face of the stopper and the interior chamber portion of the cavity of the stopper, and the first length of the neck portion of the barb of the proximal end of the plunger is smaller than the second length of the channel portion of the cavity of the stopper, when the stopper is not snap-fitted to the plunger.
Example 5 is the syringe of example 4, wherein the first length of the neck portion of the barb of the proximal end of the plunger is at least 0.3 millimeter or about 0.5 millimeter smaller than the second length of the channel portion of the cavity of the stopper.
Example 6 is the syringe of any one of examples 3 to 5, wherein the neck portion of the barb of the proximal end of the plunger has a first compression section, the channel portion of the cavity of the stopper has a second compression section, and the first compression section of the neck portion of the barb of the proximal end of the plunger is more tapering than the second compression section of the channel portion of the cavity of the stopper.
Example 7 is the syringe of example 6, wherein the first compression section of the neck portion of the barb of the proximal end of the plunger is tapering towards the head portion of the barb of the proximal end of the plunger.
Example 8 is the syringe of example 6 or 7, wherein the second compression section of the channel portion of the cavity of the stopper is tapering towards the interior chamber portion of the cavity of the stopper.
Example 9 is the syringe of any one of examples 2 to 8, comprising a gasket arranged about the barb of the proximal end of the plunger, wherein the gasket is contacting the abutting face of the proximal end of the plunger and the distal face of the stopper.
Example 10 is the syringe of any one of the preceding examples 1 to 9, wherein the distal face of the stopper has a bulge circumferentially extending around the cavity of the stopper opening at the distal face.
Example 11 is the syringe of any one of the preceding examples 1 to 10, wherein a liquid is arranged in the chamber of the barrel.

### Brief Description of the Drawings

The syringe according to the invention and the tightness testing method according to the invention are described in more detail herein below by way of an exemplary embodiment and with reference to the attached drawings, in which:
- Fig. 1: shows a schematic cross sectional side view of a first embodiment of a syringe according to the invention;
- Fig. 2: shows detail A of Fig. 1;
- Fig. 3: shows a schematic cross sectional side view of a second embodiment of a syringe according to the invention;
- Fig. 4: shows detail B of Fig. 3;
- Fig. 5: shows a schematic cross sectional side view of a third embodiment of a syringe according to the invention; and
- Fig. 6: shows detail C of Fig. 5.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

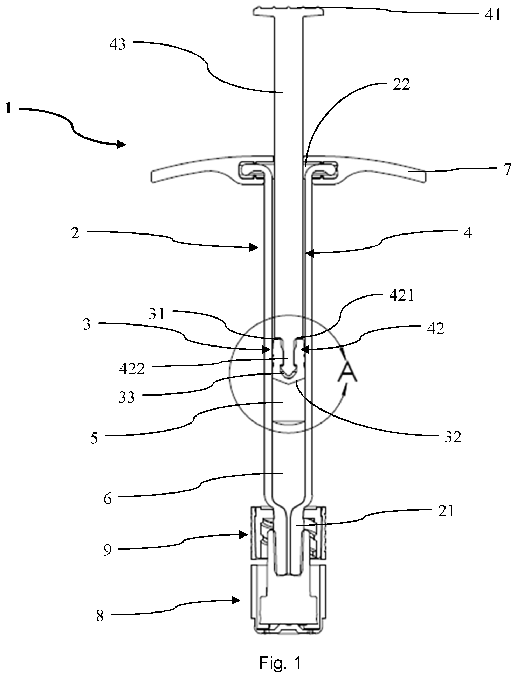

Fig. 1 shows an ophthalmic prefilled syringe (PFS) 1 as a first embodiment of a syringe according to the invention in an upright position. The PFS 1 comprises a barrel 2, a stopper 3 and a plunger 4. The barrel 2 is essentially cylindrical with a hollow interior, an orifice 21 and an opening 22 opposite to the orifice 21. The stopper 3 is placed in the hollow interior of the barrel 2 defining a sealed chamber 5 in the interior of the barrel 2. More specifically, the stopper 3 has a top distal face 31 and a bottom proximal face 32, wherein the chamber 5 is located between the proximal face 32 and the orifice 21 of the barrel 2. The orifice 21 has a spout with an internal channel tightly closed by a closure cap 8. Around the orifice 21 the PFS 1 is equipped with a Luer lock adapter 9.

Inside the chamber 5 a liquid ophthalmic drug substance 6 is arranged. The stopper 3 is displaceable in the interior of the barrel 2 thereby varying a volume of the chamber 5. More specifically, by downwardly or proximally moving the stopper 3 towards the orifice 21, the volume of the chamber 5 is reduced which allows to expel the drug substance 6 out of the orifice 21 after removal of the cap 8.

The stopper 2 is further designed with an internal cavity 33. The cavity 33 upwardly opens to the distal face 31.

The plunger 4 has a vertical cylindrical rod portion 43 extending through the opening 22 of the barrel 2 into its hollow interior. It has a top distal end 41 and a bottom proximal end 42. The distal end 41 is equipped with a top finger push surface having ribs. The bottom end 42 comprises an essentially horizontal abutting face 421 and a barb 422 downwardly extending from the abutting face 421 in a proximal direction. As described in more detail below, the barb 422 is positioned inside the cavity 33 of the stopper 3.

The PFS 1 further has an extended finger flange 7 clipped to the barrel 2 around its opening 22. The finger flange 7 allows for conveniently operating the PFS 1.

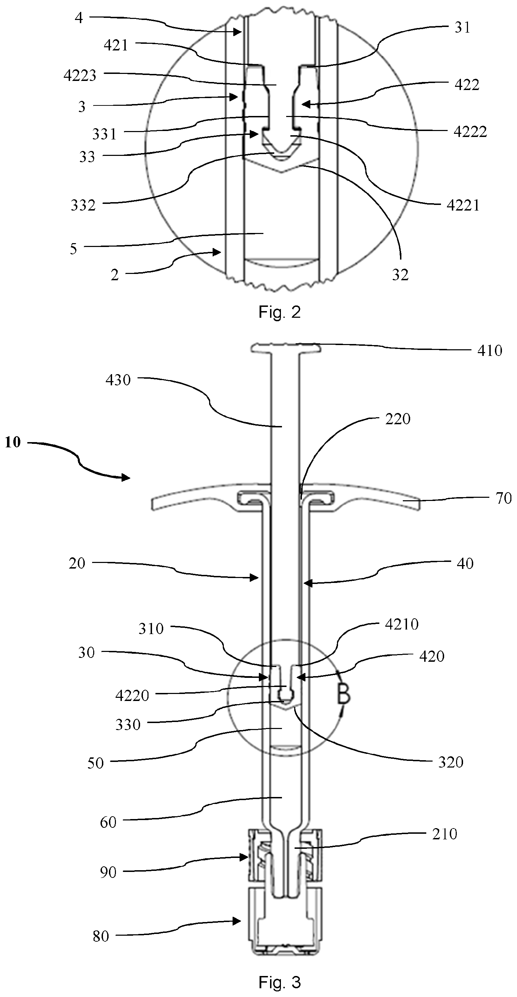

In Fig. 2, a section of the PFS 1 around the stopper 3 is shown in more detail. Thereby, it can be seen that the cavity 33 of the stopper 3 has a lower or proximal interior chamber portion 332 and an upper or distal channel portion 331 extending from the proximal face 31 to the interior chamber portion 332. The barb 422 has a proximal or lower head portion 4221 essentially corresponding to the interior chamber portion 332 of the cavity 33 and a distal or upper neck portion 4222 essentially corresponding to the channel portion 331 of the cavity 33. The stopper 3 is snapped on the barb 422 of the plunger 4 such that a form fit connection is established.

The neck portion 4222 of the barb 422 of the proximal end 42 of the plunger 4 has a conical first compression section 4223 and the channel portion 331 of the cavity 33 of the stopper 3 has a straight or cylindrical second compression section located around the first compression section 4223. Thus, the first compression section 4223 of the neck portion 4222 is downwardly or proximally more tapering than the second compression section of the channel portion 331, such that the stopper material is compressed around the first compression section 4223.

Like this, the first compression section 4223 of the neck 4222 of the barb 422 together with the second compression section of the channel 331 of the cavity 33 establish a sealing structure sealing the cavity 33 of the stopper 3 such that it is microbiologically sealed.

For verifying efficacy of the sealing structure of the PFS 1, it can be tested by an embodiment of a method of testing tightness between a stopper and a plunger in a syringe according to the invention. Thereby, the method is applied to the non-assembled PFS 1 and comprises the following steps: opening the cavity 33 of the stopper 3 to the proximal face 32, e.g., by drilling a hole into the proximal face 32 or by cutting a portion of the proximal face 32; setting the stopper 3 through the opening 22 of the barrel 2 into a hollow interior of the barrel 2 such that the distal face 31 of the stopper 3 is directed towards the plunger 4; coupling a Helium detector to the opening 22 of the barrel 2, and providing Helium through the orifice 21 of the barrel 2 at an overpressure of about 5 mbar. The Helium detector being measures a concentration of Helium distally from the stopper 3. Thus, if the Helium detector does not detect any Helium, the sealing structure of the PFS 1 closes the cavity 33 gas tight. Otherwise, closing tightness is evaluated by means of the measured Helium concentration.

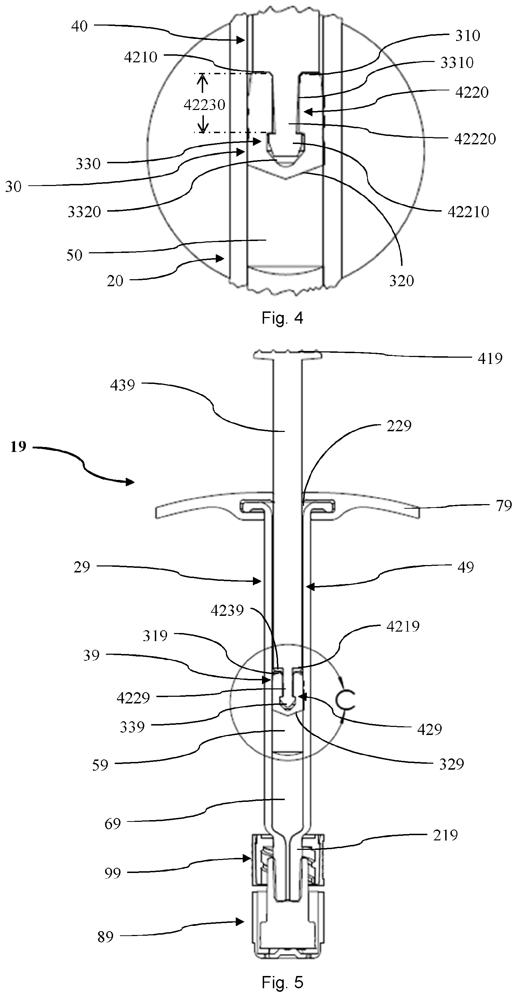

Fig. 3 and Fig. 4 show another ophthalmic PFS 10 as second embodiment of a syringe according to the invention in an upright position. The PFS 10 is widely identically embodied as the PFS 1 of Figs. 1 and 2. In particular, it identically comprises a barrel 20 with an orifice 210 and an opening 220, a stopper 30 with a distal face 310, a proximal face 320 and a cavity 330 having an interior chamber portion 3320 and a cylindrical channel portion 3310, a chamber 50, a drug substance 60, an extended finger flange 70, a cap 80 and a Luer lock adapter 90.

The plunger 40 has a vertical cylindrical rod portion 430 extending through the opening 220 of the barrel 20 into its hollow interior. It has a top distal end 410 and a bottom proximal end 420. The proximal end 420 comprises an essentially horizontal abutting face 4210 and a barb 4220 downwardly extending from the abutting face 4210 in a proximal direction.

As can be best seen in Fig. 4, the barb 4220 has a lower or proximal head portion 42210 and an upper or distal essentially cylindrical neck portion 42220. The neck portion 42220 has a first axial length 42230 extending between the abutting face 4210 and the head portion 42210. The first axial length 42230 is smaller than a second axial length of the channel portion 3310 of the cavity 330 of the stopper 30 extending between the distal face 310 of the stopper 30 and the interior chamber portion 3320 of the cavity 330. By these differing first and second lengths the stopper 30 is compressed towards abutting face 4210 of the plunger 40 when the stopper 30 is snapped on the barb 4220 of the plunger 40.

Like this, the shorter first axial length 42230 of the neck portion 42220 of the barb 4220 together with the second axial length of the channel portion 3310 of the cavity 330 of the stopper 30 establish a sealing structure. In particular, by means of this structure, the distal face 310 of the stopper 30 is compressed to the abutting face 4210 of the plunger 40 such that the cavity 330 of the stopper 30 is microbiologically sealed.

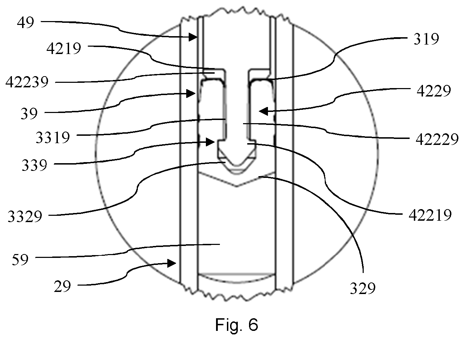

Fig. 5 and Fig. 6 show still another ophthalmic PFS 19 as third embodiment of a syringe according to the invention, in an upright position. The PFS 19 is widely identically embodied as the PFS 1 of Figs. 1 and 2 and as the PFS 10 of Figs. 3 and 4. In particular, it identically comprises a barrel 29 with an orifice 219 and an opening 229, a stopper 39 with a distal face 319, a proximal face 329 and a cavity 339 having an interior chamber portion 3329 and a cylindrical channel portion 3319, a chamber 59, a drug substance 69, an extended finger flange 79, a cap 89 and a Luer lock adapter 99.

The plunger 49 has a vertical cylindrical rod portion 439 extending through the opening 229 of the barrel 29 into its hollow interior. It has a top distal end 419 and a bottom proximal end 429. The proximal end 429 comprises an essentially horizontal abutting face 4219 and a barb 4229 downwardly extending from the abutting face 4219 in a proximal direction.

As can be best seen in Fig. 6, the barb 4229 has a lower or proximal head portion 42219 and an upper or distal essentially cylindrical neck portion 42229. The barb 4229 of the plunger 49 is snapped in the cavity 339 of the stopper 39 such that a form fit connection is established between the plunger 49 and the stopper 39. Thereby, the neck portion 42229 of the barb 4229 is located in the channel portion 3319 of the cavity 339 and the head portion 42219 of the barb 4229 in the interior chamber portion 3329 of the cavity 339.

In between the abutting face 4219 of the proximal end 429 of the plunger 49 and the distal face 319 of the stopper 39, a gasket 42239 is arranged. The gasket 42239 is ring shaped and extends around the neck portion 42229 of the barb 4229. The barb 4229 is dimensioned and shaped such that the gasket 42239 is compressed. Like this, the gasket 42239 together with the barb 4229 establishes a sealing structure. By means of this sealing structure, the distal face 310 of the stopper 30 tightened to the proximal end 429 of the plunger 49 such that the cavity 330 of the stopper 30 is microbiologically sealed.

This description and the accompanying drawings that illustrate aspects and embodiments of the present invention should not be taken as limiting-the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below. For example, it is possible to operate the invention in an embodiment wherein:
- The sealing structure of the PFS 1 of Fig. 1 and Fig. 2 is combined with the sealing structure of the PFS 10 of Figs. 3 and 4.
- The sealing structure of the PFS 1 of Fig. 1 and Fig. 2 is combined with the sealing structure of the PFS 19 of Figs. 5 and 6.

The disclosure also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. A method of testing tightness between a stopper (3; 30; 39) and a plunger (4; 40; 49) in a syringe (1; 10; 19), wherein the stopper (3; 30; 39) has a distal face (31; 310; 319), a proximal face (32; 320; 329) and an internal cavity (33; 330; 339) opening at the distal face (31; 310; 319), and the syringe (1; 10; 19) has a barrel (2; 20; 29) with a hollow interior, an orifice (21; 210; 219) and an opening (22; 220; 229) opposite to the orifice (21; 210; 219), the method comprising
opening the cavity (33; 330; 339) of the stopper (3; 30; 39) to the proximal face (32; 320; 329),
setting the stopper (3; 30; 39) through the opening (22; 220; 229) of the barrel (2; 20; 29) into a hollow interior of the barrel (2; 20; 29) such that the distal face (31; 310; 319) of the stopper (3; 30; 39) is directed towards the plunger (4; 40; 49),
coupling a gas detector to the opening (22; 220; 229) of the syringe barrel (2; 20; 29), and
providing a gas through the orifice (21; 210; 219) of the barrel (2; 20; 29).

2. The method of claim 1, further comprising operating the gas detector to detect gas distally from the stopper (3; 30; 39).

3. The method of claim 2, wherein the gas detector measures a concentration of the gas distally from the stopper (3; 30; 39).

4. The method of claim 3, comprising
evaluating tightness of the connection between the stopper (3; 30; 39) and the plunger (4; 40; 49).

5. The method of claim 3 or 4, comprising
comparing different sealing structures, and
selecting a most appropriate sealing structure.

6. The method of any one of the preceding claims, comprising generating a vacuum distally from the stopper (3; 30; 39).

7. The method of claim 6, wherein the vacuum is in a submillibar pressure range.

8. The method of claim 6 or 7, comprising
forcing gas through the stopper (3; 30; 39).

9. The method of any one of the preceding claims, wherein, when providing the gas through the orifice (21; 210; 219), it enters the cavity (33; 330; 339) of the stopper (3; 30; 39) through the opened proximal face (32; 320; 329) such that it is arranged inside the cavity (33; 330; 339) of the stopper (3; 30; 39).

10. The method of claim 9, comprising
the gas detector verifying, if the gas is not capable of exiting the cavity (33; 330; 339) of the stopper (3; 30; 39) though the distal face (31; 310; 319) of the stopper (3; 30; 39).

11. The method of any one of the preceding claims, comprising
rating the tightness by evaluating the amount of gas detected distally from the stopper (3; 30; 39).

12. The method of any one of the preceding claims, comprising
rating the tightness by evaluating the gas flow rate detected distally from the stopper (3; 30; 39).
